# EUROPEAN PATENT SPECIFICATION

(11) **EP 4 284 281 B1**
(45) Date of publication and mention of the grant of the patent: **28.01.2026**
(21) Application number: 22746786.7
(22) Date of filing: 31.01.2022
(51) Int. Cl.: A61B 18/14, A61B 18/00, A61B 18/12, A61B 18/18

(54) **MINIMALLY INVASIVE DEVICES FOR MEASURING INTESTINAL POTENTIAL DIFFERENCE**
MINIMAL INVASIVE VORRICHTUNGEN ZUR MESSUNG DER POTENTIALDIFFERENZ DES DARMS
DISPOSITIFS MINIMALEMENT INVASIFS DE MESURE DE LA DIFFÉRENCE DE POTENTIEL INTESTINALE

(30) Priority: 31.01.2021 US 202163143876 P
(43) Date of publication of application: 06.12.2023
(73) Proprietor: The General Hospital Corporation, Boston, MA 02114 (US)
(72) Inventor: TEARNEY, Guillermo, Boston, MA 02114 (US); OTUYA, David Odeke, Boston, MA 02114 (US); FARROKHI, Hamid, Boston, MA 02114 (US); SHI, Serena Qinyun Z, Boston, MA 02114 (US); SILVA, Sarah Lynn, Boston, MA 02114 (US); DONG, Jing, Boston, MA 02114 (US)
(74) Representative: Maiwald GmbH
(86) International application number: PCT/US2022/014508
(87) International publication number: WO 2022/165309

(56) References cited:
- WO-A1-88/06860
- US-A- 4 432 366
- US-A1- 2005 085 762
- US-A1- 2008 004 621
- US-A1- 2008 009 764
- US-A1- 2008 288 031
- US-A1- 2011 071 395
- US-A1- 2012 282 644
- US-A1- 2015 080 677
- US-A1- 2015 202 329
- US-A1- 2021 000 521
- US-B1- 7 182 847
- GUSTKE R F ET AL: "Human intestinal potential difference: recording method and biophysical implications.", THE JOURNAL OF PHYSIOLOGY, vol. 321, no. 1, 1 December 1981 (1981-12-01), GB, pages 571 - 582, XP093211045, ISSN: 0022-3751, DOI: 10.1113/jphysiol.1981.sp014003

## Description

### CROSS-REFERENCE TO RELATED APPLICATIONS

The present application is based on and claims priority from U.S. Patent Application Ser. No. 63/143,876, filed on January 31, 2021.

### STATEMENT REGARDING FEDERALLY SPONSORED RESEARCH

N/A

### BACKGROUND

The lining of the small intestine absorbs nutrients and water and provides a protective barrier which prevents translocation of luminal pathogens into the body. The intestinal barrier includes three layers: the mucus lining layer, the epithelial layer, and the lamina propria. Cells that make up the epithelial layer are connected by the tight junction (TJ) proteins which regulate intestinal paracellular permeability by providing selectivity to the flow of ions, small molecules, and solutes across the epithelial lining. A variety of luminal pathogens induce changes in the TJ proteins, causing increased paracellular permeability, to gain access to the lamina propria. In addition, disruption of this barrier by effacement of the epithelial lining caused by environmental or dietary factors may also result in increased intestinal permeability. The pathophysiology of many diseases can be associated with a dysfunctional intestinal barrier. For instance, studies have shown an association between barrier dysfunction and conditions such as irritable bowel disease (IBD), evidenced by an increased expression of claudin-2 protein. It is also believed that a genetic defect linked to the TJ proteins in the intestinal barrier predisposes one to Crohn's disease. Ex vivo studies have shown a doubling of the permeability of colonic biopsies from patients with irritable bowel syndrome (IBS). Changes in intestinal permeability have been linked to elevations in plasma lipopolysaccharide (LPS) involved in the onset and progression of metabolic disease. Celiac disease patients have been shown to possess defective TJ proteins and associated increased paracellular permeability. There is also increasing evidence linking increased intestinal permeability to type 1 diabetes (T1D), liver cirrhosis, primary biliary cholangitis (PBC), type 2 diabetes, nonalcoholic steatohepatitis (NASH), chronic kidney disease, and chronic heart failure (CHF).

Thus, intestinal permeability measurements could lead to insights that may improve our ability to predict who is likely to develop these diseases and help develop therapies to restore permeability and slow or even prevent them. A number of methods are currently in use for assessing intestinal permeability. The most popular method is the dual sugars test, where two probe sugars of different molecular sizes, a large molecule sugar such as lactulose (L) and a small molecule sugar such as mannitol (M), are administered orally and their ratios (L:M ratio) measured in urine. This ratio is an indicator of the level of intestinal permeability, where an increase in the amount of large molecule sugar in urine signifies increased intestinal permeability. While non-invasive in nature, this technique requires access to a facility having chromatography and mass spectroscopy capabilities in order to perform the analysis. Furthermore, urine samples may frequently be contaminated, in which case the test may be rendered useless. In addition, it can be challenging to obtain pristine urine samples from infants and children. As an alternative approach, blood biomarkers have been proposed for assessing the level of permeability in the small intestine but have not yielded much success since they can be affected by factors such as gastrointestinal motility, mucosal blood flow, and the distribution of the biomarkers in the body. Finally, the L:M ratio and serum tests provide a measure of the permeability of the small intestine as a whole but do not address variation in permeability along the intestine. Therefore, the limitations of the current methods highlight the need for minimally invasive, less expensive, and more rapid techniques for assessing small intestinal permeability. Exemplary methods for measuring intestinal potential difference are described in e.g., WO 88/06860 A1 and Gustke R.F. et al: "Human intestinal potential difference: recording method and biophysical implications", The Journal of Physiology, vol. 321, no. 1, 1 December 1981, pages 571-582.

### SUMMARY OF THE INVENTION

Accordingly, new systems for assessing small intestinal permeability are desirable.

Here, we present the development of a clinical, optical coherence tomography (OCT)-guided, trans-nasal introduction tube (TNIT)-compatible IPD measurement device. In certain embodiments, the device may include a 1.0-1.2 mm outer diameter probe terminated by an Ag/AgCl electrode and an integrated optical fiber. The device may be introduced through the lumen of the TNIT device into the subject's small intestine. Tissue-probe contact may be confirmed through M-mode OCT imaging, and IPD values may be measured with reference to subcutaneous tissue. A feasibility experiment, conducted in a Yorkshire swine in vivo, measured a baseline duodenal IPD of -12.16 ± 0.17 mV, which is consistent with the expected value. This trans-nasal, image-guided IPD probe may be suitable for assessing localized intestinal permeability in real time in unsedated subjects.

According to the present invention there is provided a system for determining intestinal potential difference, comprising: a measurement probe comprising a measurement tube having a measurement lumen which houses a measurement electrode therein,
the measurement probe further comprising a proximity sensor for determining a proximity of a distal end of the measurement tube to the intestinal tissue;
a measurement fluid delivery system in fluid communication with the measurement lumen,
the measurement fluid delivery system being configured to deliver an electrically-conductive fluid into the measurement lumen such that the electrically-conductive fluid is electrically coupled to the measurement electrode,
the measurement lumen including an outlet at a distal end thereof through which the electrically-conductive fluid exits the measurement lumen and contacts an intestinal tissue of a subject to provide electrical coupling between the measurement electrode and the intestinal tissue, and
the measurement fluid delivery system comprising a perfusion pump and perfusion tube; and
a controller coupled to the measurement electrode configured to
measure a potential difference between the measurement electrode and a reference electrode electrically coupled to the subject.

### BRIEF DESCRIPTION OF THE DRAWINGS

Various objects, features, and advantages of the disclosed subject matter can be more fully appreciated with reference to the following detailed description of the disclosed subject matter when considered in connection with the following drawings, in which like reference numerals identify like elements.

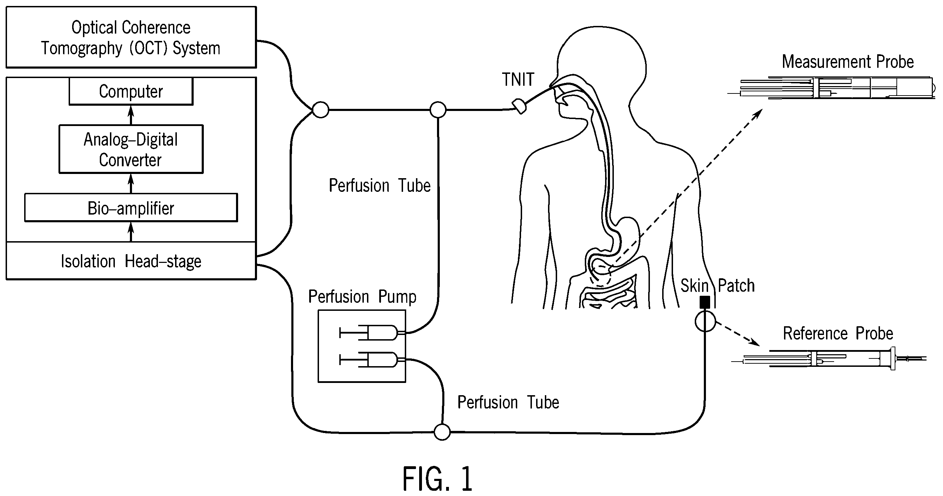

FIG. 1 shows a trans-nasal small intestinal potential difference (IPD) measuring scheme. A console, including the detection unit and data acquisition and display system, is connected to an IPD probe. The probe has a proximity sensor to provide information on small intestinal wall proximity.

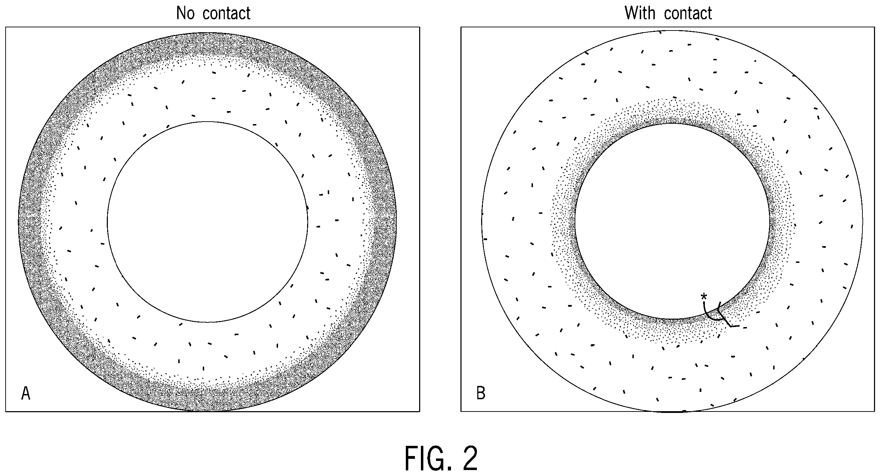

FIG. 2 shows (Panel A) an M-mode OCT image from an intestinal probe showing no contact with the intestinal wall, and (Panel B) an M-mode OCT image of an intestinal probe in contact with the intestinal wall evidenced by increased tissue scattering (seen as a darker region of the inner portion of the signal, marked using an asterisk *) nearer to the probe.

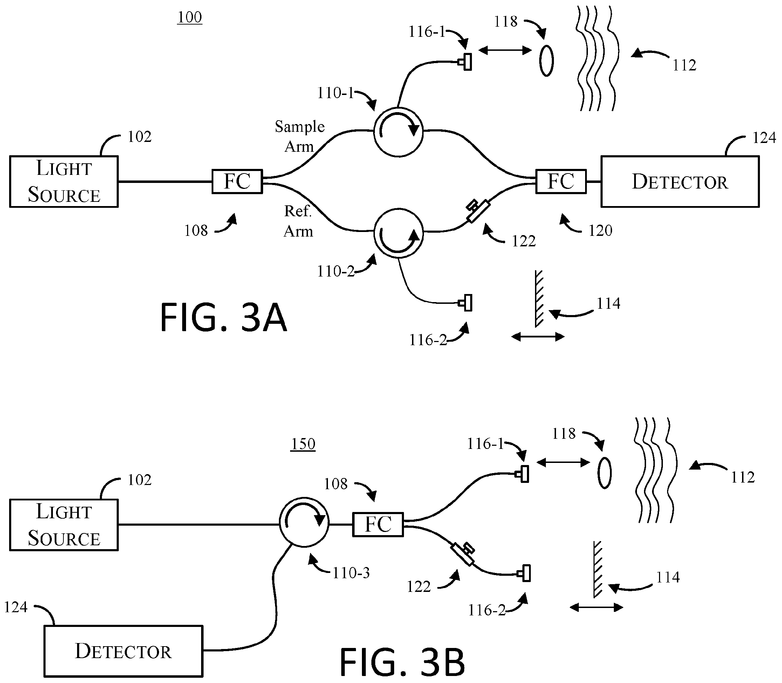

FIGS. 3A and 3B show examples of an optical coherence tomography system for use with the present system.

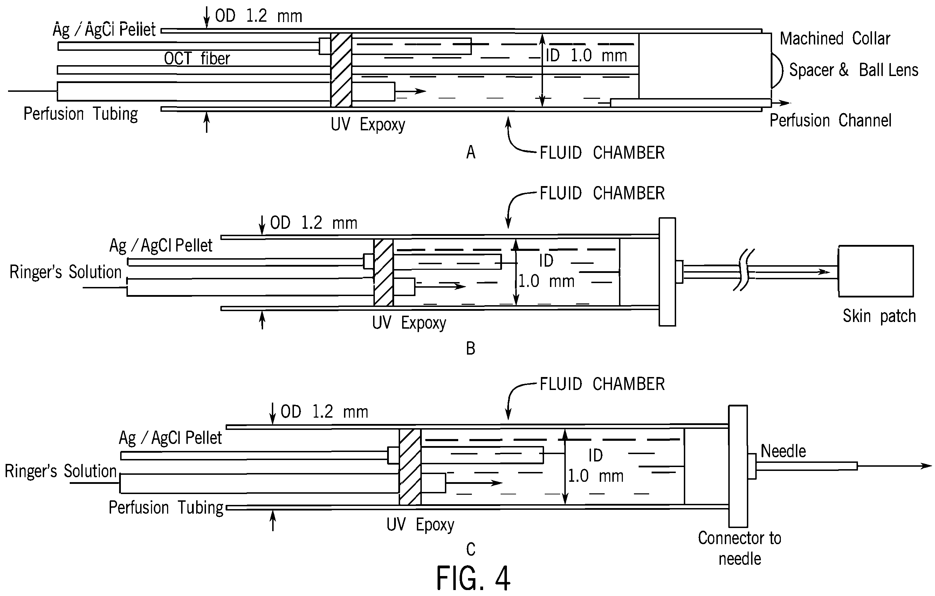

FIG. 4 shows (Panel A) a magnified view of the intestinal potential difference (IPD) measurement probe. The probe includes an Ag/AgCl electrode dipped in a mini well of lactated Ringer's solution. The mini well is continuously filled with a perfusion Ringer's solution through a micro-perfusion tube. The Ringer's solution escapes through the perfusion channel to provide ionic contact with the mucosa. An M-mode OCT probe for contact sensing includes an optical fiber that guides light to the ball lens that focuses light to mucosa; (Panels B and C) reference probes also include an Ag/AgCl electrode dipped in a mini well filled with Ringer's solution. The Ringer's solution then passes through a tube to a skin patch that is attached to abraded skin (Panel B) or through a needle into subcutaneous space (Panel C).

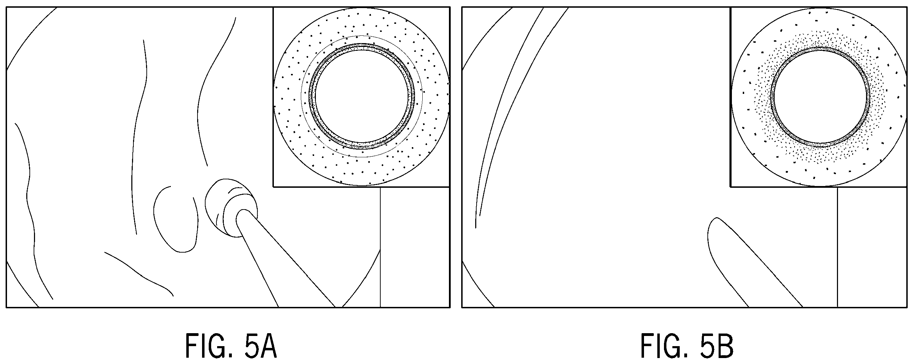

FIGS. 5A and 5B show endoscopic views of an Ag/AgCl-based measurement probe inserted into a swine duodenum. (FIG. 5A) When the probe is not in contact with tissue, an M-mode OCT dark ring is not visible (see inset). (FIG. 5B) Upon tissue contact, OCT shows a dark ring at the inner circle (see inset).

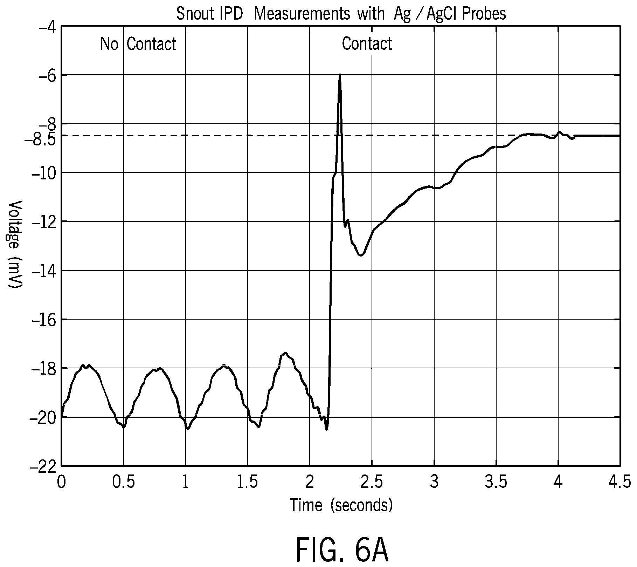

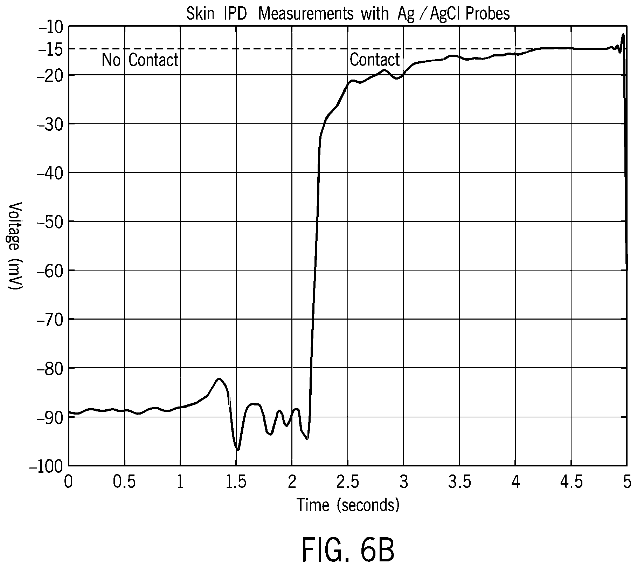

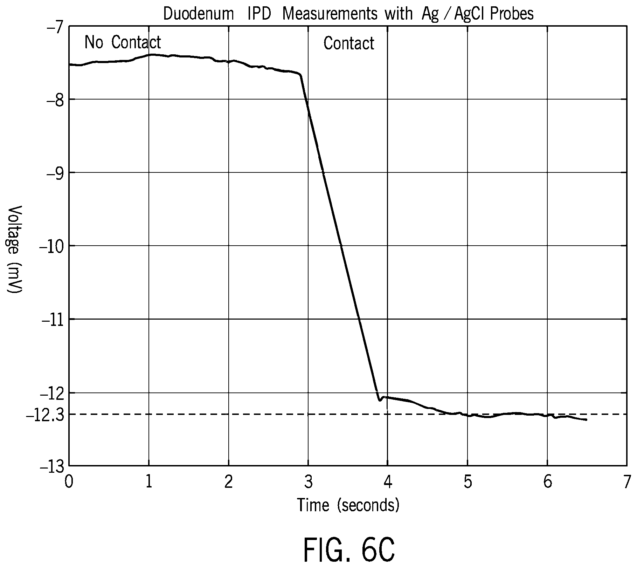

FIGS. 6A-6C show IPD measurements of (FIG. 6A) snout, (FIG. 6B) skin and (FIG. 6C) duodenum with reference to subcutaneous (SC) tissue of swine. Significant changes in IPD can be seen from no contact (yellow) to contact (green) of the probe to tissue, with -8.5 mV for snout vs SC and -15 mV for skin vs SC. The resting duodenal IPD was -12.3 mV.

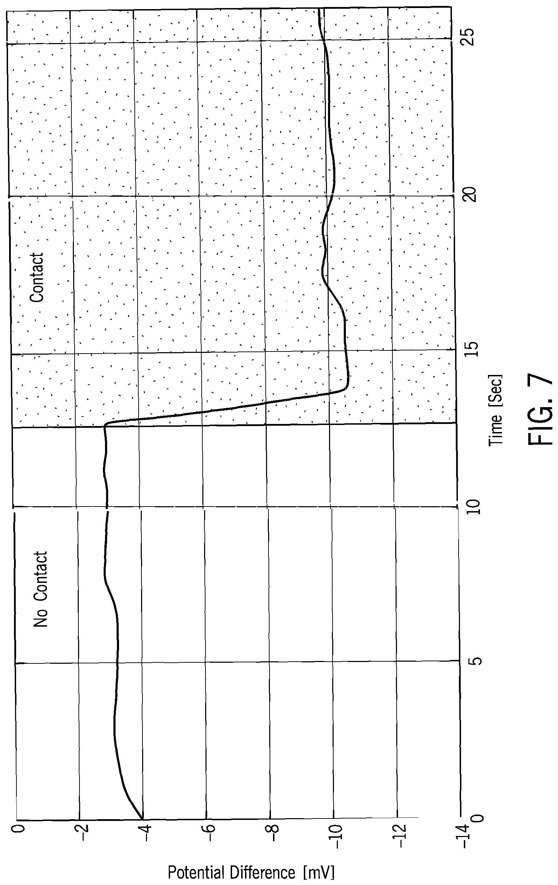

FIG. 7 shows the potential difference (in mV) obtained between the measurement and reference electrodes when the measurement probe is not in contact with the tissue (left) and when contact has been established (right), showing in this case that the potential difference drops from approximately -3 mV when the probe is not in contact to approximately -10 mV when the probe is in contact with the tissue.

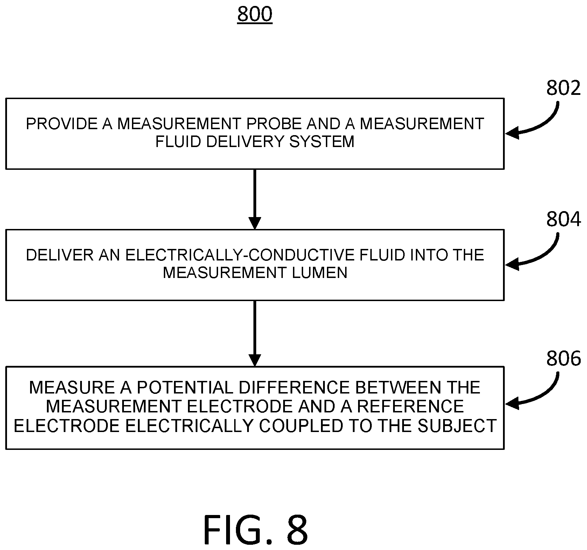

FIG. 8 shows an example of a process for determining intestinal potential difference in accordance with some embodiments of the disclosed subject matter.

### DETAILED DESCRIPTION

In accordance with some embodiments of the disclosed subject matter, mechanisms (which can include apparatus, systems, methods, and media) for assessing small intestinal permeability are provided

In various embodiments, the disclosed procedures provide trans-nasal access to the intestinal space such that no sedation, no anesthesia, and no endoscopy are required and the procedures can be conducted at low cost and low risk and the measurement probe is a reusable device. Thus, the procedures provide the ability to sample children, infants, pregnant mothers, and subjects with sedation-related issues. In various embodiments, the procedures provide contact sensing capability which ensures that the probe is in contact with the mucosa when obtaining potential difference measurements. Finally, in contrast to other procedures, the presently-disclosed procedures do not require flooding the intestine with Ringer's or other saline solution, since the proximity of the probe to the tissue can be confirmed with the proximity sensor and so only a small amount of saline is needed to maintain an ionic connection.

Certain embodiments of the procedures may be used in clinical or research settings. In clinical use, the procedures may be used to monitor responses to medications and treatments in patients with celiac disease, IBS, and other intestinal conditions. In the research setting, the procedures can be used to study the association between diet or disease or dysbiosis with intestinal permeability.

Disclosed herein are embodiments of a clinical, optical coherence tomography (OCT)-guided trans-nasal introduction tube (TNIT)-compatible intestinal potential difference (IPD) measurement device. In various embodiments, the device may include a 1.0 mm outer diameter probe terminated by an Ag/AgCl electrode and an integrated optical fiber. The device may be introduced through the lumen of the TNIT device into the subject's small intestine. Tissue-probe contact may be confirmed through M-mode OCT imaging, and IPD values are measured with reference to subcutaneous tissue. A feasibility experiment, conducted in a Yorkshire swine in vivo, measured a baseline duodenal IPD of -12.16 ± 0.17 mV, which is consistent with the expected value. Various embodiments of the trans-nasal, image guided IPD probe may be suitable for assessing localized intestinal permeability in real time in unsedated subjects.

FIG. 1 shows an embodiment of an apparatus for IPD measurement where a trans-nasal introduction tube (TNIT) is passively deployed to the small intestine. An intestinal potential difference (IPD) measurement probe (OD 1.2 m) is then threaded through the working channel of the TNIT (ID 1.5 mm). According to the present invention, the measurement probe includes a proximity sensor attached to the tip that provides information on whether the probe is in contact with intestinal mucosa. A reference probe may be connected to a patch that is placed on abraded skin. Both the measurement probe and the reference probe are continuously perfused with isotonic saline (e.g. standard Ringer's solution) to provide ionic contact between the mini electrodes embedded in the probes and the body fluids in the subject's tissue. The probes are connected to an isolation head-stage and bio-amplifier, which amplify the signal that is then digitized by an analog-digital converter and logged by a computer. Proximity sensing is useful for ensuring that the IPD probe is in contact with the intestine or fluid in the intestinal lumen.

Providing a proximity sensor helps a user (e.g. a clinician) confirm that the measurement probe is contact with the intestinal tissue as opposed to simply being near the tissue. This makes the measurement results that are obtained more consistent and reliable since the measurement electrode that is in contact with the tissue has a strong ionic coupling via the saline flowing from the end of the probe.

In certain embodiments, the proximity sensor comprises an optical coherence tomography (OCT) probe. A method of OCT imaging known as M-mode OCT can be used to give the IPD probe the functionality of proximity sensing. An optical fiber within the IPD probe guides OCT light to its distalmost end. Light from the fiber illuminates the intestine and then is scattered back, returning through the fiber to the same TNIT OCT imaging system. Although the images returned by the OCT system are circular patterns, the OCT probe at the end of the IPD probe does not rotate. Instead, the optical beam emanating from the distal end of the probe is constantly shining in one direction. The light scattered back from the tissue surface is collected by the IPD probe and guided back to the OCT system where the dark rings are created through interferometry, corresponding to the position of the tissue surface where the scattering occurs. As shown in FIG. 2, which shows images formed with no contact (FIG. 2A) or with contact (FIG. 2B), this technique allows the operator to determine whether the probe is in contact with the surrounding luminal contents or the intestine itself. By ensuring that the probe is in contact with the intestinal lining, the guided technique will prevent the measurement of incorrect potential difference values that would occur when the probe is surrounded by air, i.e. not in contact. Although the examples provided herein are directed towards use of an OCT probe, in other embodiments proximity sensing may be provided by sensors which generate impedance measurements or by pressure sensors.

FIGS. 3A and 3B show examples 100 and 150 of optical coherence tomography (OCT) systems that can be used with embodiments of the disclosed system, although other OCT systems may also be used. In either case, collimating optics 116-1 and focusing optics 118 would be housed at the distal end of the IPD reference probe such as that shown in FIG. 4A.

FIG. 3A shows a representation of a widely used configuration of an OCT system, which uses a Mach-Zehnder interferometer for OCT. FIG. 3B is another widely used configuration of an OCT system which uses a Michelson interferometer for OCT. In various embodiments the OCT system can be an SS-OCT or an SD-OCT system or a system based on other OCT modalities. In a conventional SD-OCT system, a broadband light source and a linear detector is used to acquire the entire available spectrum at a given time. The imaging depth range is determined by both the number of pixels and width of the pixels in a linear detector used to acquire the spectrum. In a conventional SS-OCT system, a wavelength-swept light source and a single point detector (such as a photodetector, photodiode, photomultiplier tube etc.) is used to acquire the spectrum as a function of time. The imaging depth range is determined by both the bandwidth of the source and the sampling rate of the detector used to acquire the spectrum.

As shown in FIG. 3A, a light source 102 can provide light to a sample arm and a reference arm via a fiber coupler 108. A portion of light is directed toward a sample arm (e.g., 80%), while a second portion of light is directed toward the reference arm (e.g., 20%). An optical circulator 110-1 directs light received from fiber coupler 108 toward a sample 112 (in the sample arm), and a second optical circulator 110-2 directs light toward a reference reflector 114 (in the reference arm). Light in the sample arm can be directed toward the sample via collimating optics 116-1 and focusing optics 118 (e.g., a lens such as the ball lens shown in FIG. 4A), which can project a beam with a depth of focus centered near the surface of sample 112. A portion of the beam can be reflected at various depths of the sample as a function of reflectivity of the sample, which is then received by focusing optics 118 and directed toward optical circulator 110-1 via collimating optics 116-1, which directs the reflected light toward a fiber coupler 120 to be combined with light from the reference arm. Collimating optics 116-2 direct a beam from the reference arm toward reference reflector 114, which reflects the beam back toward optical circulator 110-2 via collimating optics 116-2, and optical circulator 110-2 directs the light reflected by reference reflector 114 toward fiber coupler 120 via a polarization controller 122 to be combined with light from the sample arm. Fiber coupler 120 combines the light from both the sample arm and the reference arm, and directs the light toward detector 124, which generates a signal representing the structure of the sample adjacent to the probe.

System 150 depicted in FIG. 3B operates using similar principles to system 100 depicted in FIG. 3A, but includes only a single optical circulator 110-3 before fiber coupler 108, rather than using an optical circulator in both the sample arm and reference arm. In both system 100 and system 150, the length of the reference arm can be set by adjusting the position of reference reflector 114 to set the depth of the zero-delay point with respect to sample 112.

FIGS. 4A, 4B, and 4C show magnified views of IPD measurement and reference probes. The measurement probe (FIG. 4A) includes an Ag/AgCl pellet electrode dipped in a mini well of Ringer's solution. The electrode may be electrically coupled (e.g. by soldering) to a wire that is connected to the isolation head stage. A perfusion tube connected to a perfusion pump provides a continuous supply of Ringer's solution to the mini well. The Ringer's solution from the mini well seeps through a perfusion channel to the mucosa providing ionic contact between the electrode and the mucosa. An M-mode OCT probe which may include an optical fiber connected to a ball lens is used to provide contact sensing capability to the probe.

Reference probes as shown in FIGS. 4B and 4C also include an Ag/AgCl electrode bathed in a mini well (fluid chamber) of Ringer's solution. The mini well may be connected to a skin patch that is attachable to an abraded skin location (FIG. 4B) or to a needle which can be inserted into subcutaneous space (FIG. 4C). In either case, the mini well is infused with Ringer's solution through a perfusion tube connected to a syringe pump and is emitted from the end of the reference probe into either the patch or the needle to provide ionic contact with the tissue and thus an electrical path through the fluid chamber to the Ag/AgCl electrode.

In various embodiments, the construction of the measurement (FIG. 4A) and the reference (FIGS. 4B, 4C) probes may be similar. Each probe may be formed from a cylindrical tube having a relatively small outer diameter, for example 1.2 mm. In the case of the measurement electrode, the cylindrical body of the probe may be sufficiently small so as to facilitate insertion of the probe through a channel of a TNIT (see FIG. 1), which may have an inner diameter as small as 1.5 mm. Thus, the final outer diameter, shown as 1.2 mm in FIG. 4A, may be varied or adjusted depending on the conditions of a particular application including the size of the insertion channel through which the probe may be introduced into the subject.

Each probe may include a fluid chamber (FIG. 4) which is continuously filled via perfusion tubing. In addition to keeping the respective electrodes bathed in saline solution within the probe, this continuous flow of saline into the fluid chamber also causes a steady stream of saline to exit the probes to the contact regions. In the case of the measurement probe, the saline exits the fluid chamber via the perfusion channel and irrigates the adjacent region of tissue to provide an ionic coupling to the tissue which provides an electrical connection to the electrode (the Ag/AgCl pellet). In the case of the reference probe, the fluid exiting the fluid chamber of the probe may either be carried (e.g. via tubing) to a skin patch to perfuse the patch and maintain an ionic coupling to the probe (FIG. 4B) or through a needle to the subcutaneous space of the subject (FIG. 4C) to maintain an ionic coupling from the tissue to the probe.

In one embodiment the electrode, OCT fiber (for the measurement probe), and perfusion tubing may be secured into the probe at the proximal end in a fluid-tight manner, for example using a UV-curable epoxy. At the distal end of the measurement electrode, a collar (e.g. a machined collar) may be used which accommodates the perfusion tube as well as OCT optics while also providing an otherwise fluid-tight connection. At the distal end of the reference electrode, a connector may be provided which establishes a fluid-tight connection to the end of the probe body and provides access to a needle or to a tube which leads to a patch, a needle, or other mechanism for establishing an ionic connection to the subject's tissue.

In use, a user such as a clinician or technician introduces a measurement probe into a subject (e.g. a patient or other human or animal subject). The measurement probe may be introduced through the subject's nasal cavity, for example using a TNIT (see FIG. 1). The measurement probe is then fed through the esophagus and stomach to the small intestine or other portion of the GI tract to obtain measurements.

At some point before, during, or after introduction of the measurement probe into the subject, the reference electrode is also connected to the subject, for example at or near the surface of the skin. In some embodiments, the reference electrode may include a needle (FIG. 4C) which can be inserted into a subcutaneous space of the subject so that when saline flows from the tip of the needle into the tissue, an ionic connection is made and maintained between the tissue and the electrode inside the probe.

In other embodiments, the reference electrode may be connected to a skin patch (FIG. 4B) which then establishes an ionic connection to the subject's tissue. The skin patch may be attached over an abraded skin surface to allow saline from the probe to penetrate the epidermis and establish an ionic connection with the underlying dermis and tissue. In some embodiments the skin patch may be similar to those used for attaching biomedical skin electrodes, e.g. for conducting an electrocardiogram on the subject, and may include an abrasive material as part of the patch to abrade the skin in order to facilitate the formation of an electrical connection with the dermis or subdermal tissue. In various embodiments the skin patch may be adapted for receiving a flow of saline from the reference probe, including having an input for tubing which carries the saline from the electrode to the patch. In certain embodiments the patch may be relatively impermeable to fluid on the outer portion and may include a fluid-absorbent material on the inner portion so as to retain saline adjacent to the abraded skin and thereby promote an ionic connection to the subject's tissue. In various embodiments the skin patch may be attached to the surface of the subject's body, for example on an arm or leg region.

Once the measurement probe has been introduced into the subject's small intestine or other GI region and the reference electrode has been coupled to the subject's skin surface or subcutaneous region and an ionic connection has been established, the position of the measurement probe can be determined using the proximity sensor. In one embodiment, the measurement probe may include an OCT system as a proximity sensor (see FIGS. 1-3). The OCT system may operate in a forward-facing manner as shown in FIG. 4A such that electromagnetic radiation is emitted from the distal end of the measurement probe onto the nearby tissue (FIGS. 3A, 3B). By viewing images obtained from the OCT system (FIGS. 2A, 2B), the user can determine whether the end of the probe is (FIG. 2B) or is not (FIG. 2A) in contact with the GI tissue, for example based on the presence of a darker inner region of the interference signal that is seen when the probe is in contact with the tissue (see FIG. 2B, where the darker inner region of the signal is identified with an asterisk, '*'; see also FIG. 5A, where there is no contact with the tissue, and FIG. 5B, where the measurement probe is in contact with the tissue).

As noted previously, saline solution may be flowed into each of the probes throughout the procedure to ensure that the respective electrodes (e.g. Ag/AgCl electrodes) are bathed in saline and thus maintain an electrical contact with the solution and the environment adj acent to the probe. After the reference electrode has been placed in or on the skin surface of the subject and the measurement electrode has been guided to a suitable location within the GI tract (typically in contact with GI tissue, as determined through the use of a proximity sensor), electrical measurements may obtained between the measurement probe and the reference probe.

FIGS. 5A and 5B show endoscopic views of the IPD probe not in contact (FIG. 5A) with the mucosa and when in contact (FIG. 5B) with mucosa and the corresponding M-mode OCT images in respective insets. As seen from the M-mode OCT images, the operator can clearly ascertain when the probe is in contact with the mucosa in a non-endoscopic procedure.

FIGS. 6A-6C show the potential difference measurement curves using the IPD probe. FIG. 6A shows PD values of swine snout, FIG. 6B shows PD value of swine skin, and FIG. 6C shows IPD values of swine duodenum. In each case there is a clear difference in voltage that is recorded when the probe is contact with the particular tissue.

When the measurement probe is not in contact with the GI tissue, the potential difference is relatively lower (FIG. 7, left), i.e. is closer to 0 mV, whereas when contact is made (e.g. established using a proximity sensor such as M-mode OCT) the measured potential difference increases (i.e. becomes more negative; see FIG. 7, right) from about -3 mV in the absence of contact to about -10 mV when the probe is in contact with the tissue. When the paracellular permeability of the GI tissue increases, the potential difference is lower (i.e. less negative and closer to 0 mV) compared to intact tissue. In typical intestinal tissue with intact TJs, the potential difference is expected to be in a range of -8 mV to -15 mV, whereas tissues with lower potential differences (e.g. values of -5 mV or lower) may be considered potentially leaky and should be further investigated. Thus, by obtaining measurements of GI tissue it is possible to establish whether the tight junctions of the epithelium in a particular region are healthy and intact or are compromised and permeable. Furthermore, regional differences in tissue integrity can be determined by moving the measurement probe along the length of a particular section of the GI tract, which can obtain more granular information regarding the integrity of the GI tissue and help diagnose conditions.

Thus, disclosed herein are embodiments of a robust, TNIT-compatible IPD probe system that is minimally invasive, cost-efficient, and provides localized, real-time intestinal potential difference measurement. The IPD probe measured a consistent duodenum IPD of -12.16 ± 0.17 mV, which is close to the literature value of -12 ± 1.3 m V and an improvement from agar probe measurements. In various embodiments, the IPD probe includes OCT capabilities to perform M-mode OCT imaging which has proved to be a reliable method for contact-sensing. Various embodiments of the IPD probe may be used for diagnosis and monitoring of diseases that affect intestinal permeability. In certain embodiments, the system may include a skin patch for electrically coupling the reference probe to the subject's body which greatly increases the minimally-invasive profile of the system.

FIG. 8 shows an example 800 of a process for determining intestinal potential difference in accordance with some embodiments of the disclosed subject matter. As shown in FIG. 8, at 802, process 800 can provide a measurement probe and a measurement fluid delivery system. The measurement probe may include a measurement tube having a measurement lumen which houses a measurement electrode therein, where the measurement fluid delivery system may be in fluid communication with the measurement lumen. At 804, process 800 can deliver an electrically-conductive fluid into the measurement lumen. The electrically-conductive fluid may be delivered using the measurement fluid delivery system. Delivering the electrically-conductive fluid in the measurement lumen may ensure that the fluid is electrically coupled to the measurement electrode. The measurement lumen may include an outlet at a distal end thereof through which the electrically-conductive fluid exits the measurement lumen and contacts an intestinal tissue of a subject to provide electrical coupling between the measurement electrode and the intestinal tissue. Finally, at 806, process 800 can measure a potential difference between the measurement electrode and a reference electrode electrically coupled to the subject. Measuring a potential difference may be performed using a controller coupled to the measurement electrode.

It should be understood that the above described steps of the process of FIG. 8 can be executed or performed in any order or sequence not limited to the order and sequence shown and described in the figures. Also, some of the above steps of the processes of FIG. 8 can be executed or performed substantially simultaneously where appropriate or in parallel to reduce latency and processing times.

## Claims

1. A system for determining intestinal potential difference, comprising:
a measurement probe comprising a measurement tube having a measurement lumen which houses a measurement electrode therein,
the measurement probe further comprising a proximity sensor (100,150) for
determining a proximity of a distal end of the measurement tube to the intestinal tissue;
a measurement fluid delivery system in fluid communication with the measurement lumen,
the measurement fluid delivery system being configured to deliver an electrically-conductive fluid into the measurement lumen such that the electrically-conductive fluid is electrically coupled to the measurement electrode,
the measurement lumen including an outlet at a distal end thereof through which the electrically-conductive fluid exits the measurement lumen and contacts an intestinal tissue of a subject to provide electrical coupling between the measurement electrode and the intestinal tissue, and
the measurement fluid delivery system comprising a perfusion pump and perfusion tube; and
a controller coupled to the measurement electrode configured to measure a potential difference between the measurement electrode and a reference electrode electrically coupled to the subject.

2. The system of claim 1, wherein the proximity sensor comprises an optical fiber disposed within the measurement tube,
wherein a distal end of the optical fiber is configured to emit an electromagnetic radiation from the distal end of the measurement tube.

3. The system of claim 2, wherein the optical fiber of the proximity sensor comprises an optical coherence tomography, OCT, system,
wherein the optical fiber comprises a sample arm of the OCT system, and
wherein the OCT system further comprises a reference arm.

4. The system of claim 3, wherein the OCT system further comprises optics coupled to the distal end of the optical fiber for focusing light onto the intestinal tissue and receiving light backscattered from the intestinal tissue.

5. The system of claim 1, wherein the reference electrode is electrically coupled to a tissue of the subject.

6. The system of claim 5, wherein the reference electrode is housed within a reference probe,
wherein the reference tube comprises a reference lumen which houses the reference electrode therein.

7. The system of claim 6, wherein the reference probe further comprises a reference fluid delivery system in communication with the reference lumen,
wherein the reference fluid delivery system is configured to deliver an electrically-conductive fluid into the reference lumen such that the electrically-conductive fluid is electrically coupled to the reference electrode, and
wherein the reference lumen includes an outlet at a distal end thereof through which the electrically-conductive fluid exits the reference lumen and contacts the tissue to provide electrical coupling between the reference electrode and the tissue.

8. The system of claim 7, wherein the reference probe further comprises at least one of a skin patch or a needle fluidly coupled to the reference lumen by the electrically-conductive fluid,
wherein the skin patch or needle is configured to contact the tissue such that the tissue is electrically coupled to the reference electrode by the electrically-conductive fluid.

9. The system of claim 8, wherein the skin patch comprises an abrasive substance to abrade an adjacent region of skin of the subject.

10. The system of any one of claims 1-9, wherein the controller is coupled to the measurement electrode and the reference electrode through an isolation head-stage and a bio-amplifier for receiving electrical signals acquired by the measurement electrode and reference electrode,
and wherein the controller comprises a processor configured to determine from the received signals the potential difference between the measurement electrode and the reference electrode.

11. The system of any one of claims 1-9, wherein the measurement electrode comprises an Ag/AgCl electrode.

12. The system of any one of claims 1-9,
wherein the electrically-conductive fluid comprises a saline solution.

13. The system of any one of claims 1-9, wherein the measurement probe is configured to be inserted into the subject using a trans-nasal introduction tube, TNIT.

## Patentansprüche

1. System zum Bestimmen einer intestinalen Potentialdifferenz, umfassend:
eine Messsonde, die ein Messrohr umfasst, das ein Messlumen aufweist, in dem eine Messelektrode untergebracht ist,
wobei die Messsonde des Weiteren einen Näherungssensor (100, 150) zum Bestimmen einer Nähe eines distalen Endes des Messrohres zum Intestinalgewebe umfasst;
ein Messfluidzufuhrsystem in Strömungsverbindung mit dem Messlumen,
wobei das Messfluidzufuhrsystem dazu eingerichtet ist, ein elektrisch leitfähiges Fluid in das Messlumen zuzuführen, dergestalt, dass das elektrisch leitfähige Fluid elektrisch mit der Messelektrode gekoppelt ist,
wobei das Messlumen an seinem distalen Ende einen Auslass aufweist, durch den das elektrisch leitfähige Fluid aus dem Messlumen austritt und ein Intestinalgewebe eines Patienten kontaktiert, um eine elektrische Kopplung zwischen der Messelektrode und dem Intestinalgewebe bereitzustellen, und
wobei das Messfluidzufuhrsystem eine Perfusionspumpe und einen Perfusionsschlauch umfasst; und
eine Steuerung, die mit der Messelektrode gekoppelt und dazu eingerichtet ist, eine Potentialdifferenz zwischen der Messelektrode und einer Referenzelektrode, die elektrisch mit dem Patienten gekoppelt ist, zu messen.

2. System nach Anspruch 1, wobei der Näherungssensor eine optische Faser umfasst, die innerhalb des Messrohres angeordnet ist,
wobei ein distales Ende der optischen Faser dazu eingerichtet ist, eine elektromagnetische Strahlung von dem distalen Ende des Messrohres auszusenden.

3. System nach Anspruch 2, wobei die optische Faser des Näherungssensors ein optisches Kohärenztomographie(OCT)-System umfasst,
wobei die optische Faser einen Probenarm des OCT-Systems umfasst, und
wobei das OCT-System des Weiteren einen Referenzarm umfasst.

4. System nach Anspruch 3, wobei das OCT-System des Weiteren eine Optik umfasst, die mit dem distalen Ende der optischen Faser gekoppelt ist, um Licht auf das Intestinalgewebe zu fokussieren und von dem Intestinalgewebe zurückgestreutes Licht zu empfangen.

5. System nach Anspruch 1, wobei die Referenzelektrode elektrisch mit einem Gewebe des Patienten gekoppelt ist.

6. System nach Anspruch 5, wobei die Referenzelektrode in einer Referenzsonde untergebracht ist,
wobei das Referenzrohr ein Referenzlumen umfasst, in dem die Referenzelektrode untergebracht ist.

7. System nach Anspruch 6, wobei die Referenzsonde des Weiteren ein Referenzfluidzufuhrsystem in Kommunikation mit dem Referenzlumen umfasst,
wobei das Referenzfluidzufuhrsystem dazu eingerichtet ist, ein elektrisch leitfähiges Fluid in das Referenzlumen zuzuführen, dergestalt, dass das elektrisch leitfähige Fluid elektrisch mit der Referenzelektrode gekoppelt ist, und
wobei das Referenzlumen an seinem distalen Ende einen Auslass aufweist, durch den das elektrisch leitfähige Fluid aus dem Referenzlumen austritt und das Gewebe kontaktiert, um eine elektrische Kopplung zwischen der Referenzelektrode und dem Gewebe bereitzustellen.

8. System nach Anspruch 7, wobei die Referenzsonde des Weiteren mindestens eines von einem Hautpflaster und einer Nadel umfasst, das/die durch das elektrisch leitfähige Fluid fluidisch mit dem Referenzlumen gekoppelt ist,
wobei das Hautpflaster oder die Nadel dazu eingerichtet ist, das Gewebe zu kontaktieren, dergestalt, dass das Gewebe durch das elektrisch leitfähige Fluid elektrisch mit der Referenzelektrode gekoppelt ist.

9. System nach Anspruch 8, wobei das Hautpflaster eine abrasive Substanz umfasst, um eine benachbarte Hautregion des Patienten abzuschleifen.

10. System nach einem der Ansprüche 1-9, wobei die Steuerung über eine Isolationskopfstufe und einen Bioverstärker mit der Messelektrode und der Referenzelektrode gekoppelt ist, um elektrische Signale zu empfangen, die durch die Messelektrode und die Referenzelektrode erfasst werden,
und wobei die Steuerung einen Prozessor umfasst, der dazu eingerichtet ist, aus den empfangenen Signalen die Potentialdifferenz zwischen der Messelektrode und der Referenzelektrode zu bestimmen.

11. System nach einem der Ansprüche 1-9, wobei die Messelektrode eine Ag/AgCl-Elektrode umfasst.

12. System nach einem der Ansprüche 1-9,
wobei das elektrisch leitfähige Fluid eine Kochsalzlösung umfasst.

13. System nach einem der Ansprüche 1-9, wobei die Messsonde dazu eingerichtet ist, unter Verwendung einer transnasalen Magensonde (TNIT) in den Patienten eingeführt zu werden.

## Revendications

1. Système pour déterminer une différence de potentiel intestinal, comprenant :
une sonde de mesure comprenant un tube de mesure doté d'une lumière de mesure qui abrite à l'intérieur une électrode de mesure,
la sonde de mesure comprenant en outre un capteur de proximité (100,150) pour déterminer une proximité d'une extrémité distale du tube de mesure au tissu intestinal ;
un système de distribution du fluide de mesure en communication fluidique avec la lumière de mesure,
le système de distribution de fluide de mesure étant configuré pour distribuer un fluide électriquement conducteur dans la lumière de mesure, de sorte que le fluide électriquement conducteur soit électriquement couplé à l'électrode de mesure,
la lumière de mesure comprenant une sortie à une extrémité distale à travers laquelle le fluide électriquement conducteur sort de la lumière de mesure et entre en contact avec un tissu intestinal d'un sujet afin de fournir un couplage électrique entre l'électrode de mesure et le tissu intestinal, et
le système de distribution du fluide de mesure comprenant une pompe à perfusion et un tube de perfusion ; et
un dispositif de commande couplé à l'électrode de mesure configuré pour mesurer une différence de potentiel entre l'électrode de mesure et une électrode de référence électriquement couplée au sujet.

2. Système selon la revendication 1, dans lequel le capteur de proximité comprend une fibre optique disposée à l'intérieur du tube de mesure,
dans lequel une extrémité distale de la fibre optique est configurée pour émettre un rayonnement électromagnétique depuis l'extrémité distale du tube de mesure.

3. Système selon la revendication 2, dans lequel la fibre optique du capteur de proximité comprend un système de tomographie par cohérence optique, OCT,
dans lequel la fibre optique comprend un bras échantillon du système OCT, et dans lequel le système OCT comprend en outre un bras de référence.

4. Système selon la revendication 3, dans le système OCT comprend en outre des optiques couplées à l'extrémité distale de la fibre optique pour focaliser de la lumière sur le tissu intestinal et recevoir de la lumière rétrodiffusée depuis le tissu intestinal.

5. Système selon la revendication 1, dans lequel l'électrode de référence est électriquement couplée à un tissu du sujet.

6. Système selon la revendication 5, dans lequel l'électrode de référence est abritée dans une sonde de référence,
dans lequel le tube de référence comprend une lumière de référence qui abrite à l'intérieur l'électrode de référence.

7. Système selon la revendication 6, dans lequel la sonde de référence comprend en outre un système de distribution du fluide de référence en communication avec la lumière de référence,
dans lequel le système de distribution du fluide de référence est configuré pour fournir un fluide électrique conducteur dans la lumière de référence de sorte que le fluide électriquement conducteur soit électriquement couplé à l'électrode de référence, et
dans lequel la lumière de référence comprend une sortie à une extrémité distale correspondante à travers laquelle le fluide électriquement conducteur sort de la lumière de référence et entre en contact avec le tissu pour fournir un couplage électrique entre l'électrode de référence et le tissu.

8. Système selon la revendication 7, dans lequel la sonde de référence comprend en outre au moins un élément parmi un patch transdermique ou une aiguille couplée fluidiquement à la lumière de référence par le fluide électriquement conducteur,
dans lequel le patch transdermique ou l'aiguille est configuré(e) pour entrer en contact avec le tissu de sorte que le tissu soit électriquement couplé à l'électrode de référence par le fluide électriquement conducteur.

9. Système selon la revendication 8, dans lequel le patch transdermique comprend une substance abrasive pour abraser une région adjacente de la peau du sujet.

10. Système selon l'une quelconque des revendications 1 à 9, dans lequel le dispositif de commande est couplé à l'électrode de mesure et à l'électrode de référence à travers une phase de tête d'isolement et un bio-amplificateur pour recevoir des signaux électriques acquis par l'électrode de mesure et l'électrode de référence,
et dans lequel le dispositif de commande comprend un processeur configuré pour déterminer à partir des signaux reçus la différence de potentiel entre l'électrode de mesure et l'électrode de référence.

11. Système selon l'une quelconque des revendications 1 à 9, dans lequel l'électrode de mesure comprend une électrode Ag/AgCl.

12. Système selon l'une quelconque des revendications 1 à 9, dans lequel le fluide électriquement conducteur comprend une solution saline.

13. Système selon l'une quelconque des revendications 1 à 9, dans lequel la sonde de mesure est configurée pour être insérée dans le sujet en utilisant un tube d'introduction trans-nasal, TNIT.
